(19) Europäisches Patentamt
European Patent Office
Office européen des brevets

(11) EP 2 357 165 A1

(12) **EUROPEAN PATENT APPLICATION**
published in accordance with Art. 153(4) EPC

(43) Date of publication:
**17.08.2011 Bulletin 2011/33**

(21) Application number: **09823545.0**

(22) Date of filing: **26.10.2009**

(51) Int Cl.:
***C07C 211/40*** *(2006.01)* ***A61K 31/137*** *(2006.01)*
***A61K 31/138*** *(2006.01)* ***A61K 31/145*** *(2006.01)*
***A61K 31/47*** *(2006.01)* ***A61P 9/10*** *(2006.01)*
***A61P 25/00*** *(2006.01)* ***A61P 25/14*** *(2006.01)*
***A61P 25/16*** *(2006.01)* ***A61P 25/28*** *(2006.01)*
***A61P 43/00*** *(2006.01)* ***C07C 217/16*** *(2006.01)*
***C07C 217/20*** *(2006.01)* ***C07C 217/62*** *(2006.01)*
***C07C 323/25*** *(2006.01)* ***C07D 215/06*** *(2006.01)*

(86) International application number:
**PCT/JP2009/068340**

(87) International publication number:
**WO 2010/050435 (06.05.2010 Gazette 2010/18)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **27.10.2008 JP 2008276045**

(71) Applicant: **M's Science Corporation**
**Hyogo 650-0047 (JP)**

(72) Inventors:
- **MITA, Shiro**
  **Nishinomiya-shi**
  **Hyogo 662-0061 (JP)**
- **KOBAYASHI, Naoyuki**
  **Nara-shi**
  **Nara 631-0032 (JP)**

(74) Representative: **Vossius & Partner**
**Siebertstrasse 4**
**81675 München (DE)**

(54) **CYCLOHEXYLAMINE DERIVATIVE CONTAINING PHENYL GROUP, AND THERAPEUTIC AGENT FOR DISEASES ACCOMPANIED BY CENTRAL NERVOUS SYSTEM DISORDERS**

(57) Disclosed is a novel cyclohexylamine derivative containing a phenyl group, which is useful as a novel sigma receptor ligand. Also disclosed is a therapeutic agent for diseases accompanied by a central nervous system disorder, which comprises the compound as an active ingredient. The therapeutic agent comprises a compound represented by general formula [1] or a pharmacologically acceptable salt thereof as an active ingredient:

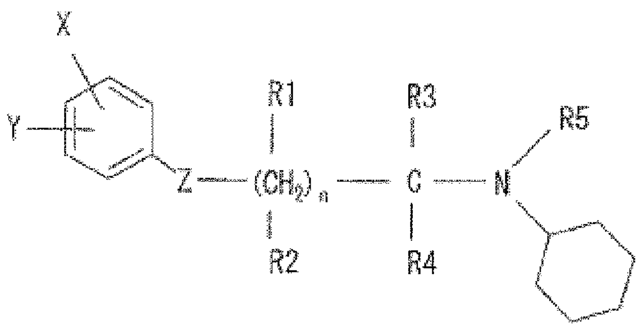

[1]

wherein X and Y independently represent a hydrogen atom, a halogen atom, a trihalomethyl group, a dihalomethyl group, a monohalomethyl group, a methoxy group, a hydroxyl group, or an alkyl group having 1 to 3 carbon atoms; R1, R2, R3 and R4 independently represent a hydrogen atom, a hydroxyl group, an alkoxyl group having 1 to 3 carbon atoms, or an alkyl group having 1 to 3 carbon atoms; R5 represents a hydrogen atom, an alkyl group having 1 to 5 carbon atoms, or an alkylene group which has 2 to 4 carbon atoms and can bind to a carbon atom other than carbon atoms that bind

to a nitrogen atom on the cyclohexyl ring to form a ring; n represents an integer of to 5; and represents a sulfur atom, a carbon atom, or an oxygen atom.

## Description

TECHNICAL FIELD

The present invention relates to a novel cyclohexylamine derivative containing a phenyl group which is useful as a novel sigma receptor ligand, and a therapeutic agent for a disease accompanied by a central nervous system disorder containing the compound as an active ingredient. More particularly, it relates to a novel cyclohexylamine derivative containing a phenyl group; a pharmaceutical composition for treatment or prevention of symptoms of the disease, containing the compound as an active ingredient; and a method for treatment or prevention of a disease accompanied by a central nervous system disorder comprising administering the active ingredient.

BACKGROUND ART

Sigma receptor ligands are known to play a variety of physiological functions by acting on sigma receptors present in central neural cells, etc. Two subtypes of the sigma receptors are currently known, and, among them, a sigma-1 receptor is known to be mainly accompanied by the central nervous system from studies such as pharmacological studies, ethological studies. Recently, it is known that the sigma-1 receptor is involved in various central nervous system disorders including schizophrenia, dementia, repression, etc. It is expected that a compound having a strong affinity to sigma receptors is useful as a therapeutic agent for treatment of a disease accompanied by a central nervous system disorder (for example, schizophrenia, dementia, depression, Parkinson's disease, Huntington's disease, and multiple sclerosis).

According to the recent studies, a finding has been obtained that pharmacological effects related to sigma receptors are based on the promotion of regeneration/maturation of the nerve tissue in association with the sigma receptors. In other words, for example, Patent Document 1 suggests that, in cerebral infarction model rats artificially created by ligating the middle cerebral artery to stop the blood flow, sigma receptors are involved in motor function recovery from cranial neuropathy, as well as suggests that compounds showing a strong affinity to sigma receptors may be useful as a therapeutic agent for the central nervous system disorder accompanied by cerebral infarction, cerebral contusion, spinal cord injury, etc.

It is known that an N-(methyl, ethyl, or propyl)-N-substituted-cyclohexylamine derivative acts as a sigma receptor ligand. As such a compound, for example, N-ethyl-N-(2-phenylethyl)cyclohexylamine derivatives (e.g., N-ethyl-N-[2-(3,4-dichlorophenylethyl)]-2-(1-pyrrolidinyl)c cyclohexylamine, etc.) are described in Patent Document 2. These compounds each have a structure such that a 1-pyrrolidinyl group is bound to an N-substituted cyclohexylamine ring. However, Patent Document 2 discloses neither a cyclohexylamine derivative having a phenylthio group nor a cyclohexylamine derivative containing a phenyl group that has one amine structure bound to a cyclohexyl ring. Moreover, there are not many known compounds that have a high affinity to sigma receptors and promote the regeneration/maturation of the central neural cells as well.

PRIOR ART DOCUMENTS

Patent Documents

Patent Document 1: US patent application publication No. 2005/0020483
Patent document 2: US patent No. 5739158

SUMMARY OF THE INVENTION

PROBLEMS TO BE SOLVED BY THE INVENTION

In view of the current situation described above, the problem of the present invention is to provide a novel cyclohexylamine derivative containing a phenyl group which is useful as a novel sigma receptor ligand, and a therapeutic agent for a disease accompanied by a central nervous system disorder containing the compound as an active ingredient. Among others, the problem of the present invention is to provide a novel compound that has a high affinity to sigma receptors and promote the regeneration and maturation of central neural cells, as well as to provide a method for treatment or prevention of the disease.

MEANS TO SOLVE THE PROBLEMS

As a result of intensive investigations on compounds in order to solve the above problems, the present inventor has discovered a series of novel compounds which are considered to be a sigma receptor agonist; said compounds have a common feature with a structure corresponding to a phenyl group and a cyclohexylamine group or a derived group thereof. That is, the present invention relates to a compound represented by the following general formula (1) or a pharmacologically acceptable salt thereof.

(Chemical formula 1)

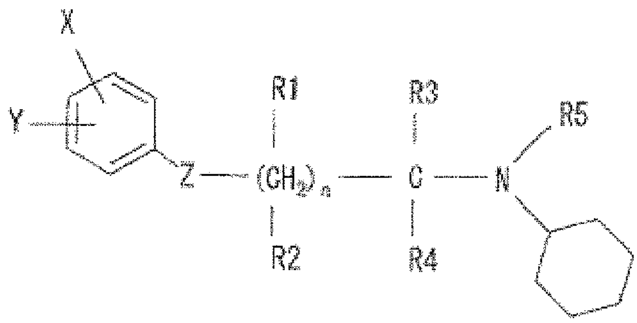

[1]

In the formula, X and Y each independently represents a hydrogen atom, a halogen atom, a trihalomethyl group, a dihalomethyl group, a monohalomethyl group, a methoxy group, a hydroxyl group, or an alkyl group having 1 to 3 carbon atoms; R1, R2, R3 and R4 each independently represents a hydrogen atom, a hydroxyl group, an alkoxyl group having 1 to 3 carbon atoms, or an alkyl group having 1 to 3 carbon atoms, provided that when a plurality of R1 and R2 are present, they may be the same or different from each other; R5 represents a hydrogen atom, an alkyl group having 1 to 5 carbon atoms, or an alkylene group which has 2 to 4 carbon atoms and binds to a carbon atom other than a carbon atom bound to a nitrogen atom on the cyclohexyl ring to form a ring; n represents an integer of 2 to 6; and Z represents a sulfur atom, a carbon atom, or an oxygen atom.

In one aspect of the present invention, the above-mentioned compound is any one of the following compounds [2] to [12], or a pharmacologically acceptable salt thereof:

[2] N-(2-(phenylthio)ethyl)-N-ethylcyclohexylamine,
[3] N-(2-phenylthio)ethyldecahydroquinoline,
[4] N-[2-(3-trifluoromethyl)phenylthio]ethyl-N-ethyloyclohexyla mine,
[5] N-[2-(3-difluoromethyl)phenylthio]ethyl-N-ethylcyclohexylam ine,
[6] N-[2-(3,4-dimethoxy)phenylthio]ethyl-N-ethylcyclohexylamine
[7] N-[2-(4-fluorophenylthio)ethyl]-N-ethyloyclohexylamine,
[8] N-(3-phenylpropyl)-N-ethylcyclohexylamine,
[9] N-[3-(4-methoxyphenyl)propyl]-N-ethylcyclohexylamine,
[10] N-[3-(4-fluorophenyl)propyl]-N-ethylcyclohexylamine,
[11] N-ethyl-N-[2-(4-fluorophenoxy)ethyl]cyclohexylamine, and
[12] N-ethyl-N-(2-phenoxyethyl)cyclohexylamine.

Also, the present invention is directed to a pharmaceutical composition for treatment or prevention of a disease accompanied by a central nervous system disorder, containing at least one of a compound represented by the following general formula [1'] and a pharmacologically acceptable salt thereof as an active ingredient.

(Chemical formula 2)

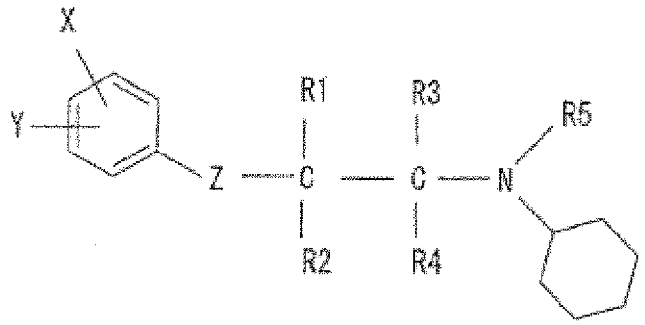

[1']

One aspect of the present invention includes at least one of the compounds [2], [3], [5], and [8], and pharmacologically acceptable salts thereof as an active ingredient.

Another aspect of the present invention is a method for treatment or prevention of a disease accompanied by a central nervous system disorder, characterized by administering a therapeutically or preventively effective amount of at least one of the compound represented by the general formula [1] and a pharmacologically acceptable salt thereof, for the disease accompanied by a central nervous system disorder, to a subject in need of the treatment or prevention. Another further aspect of the present invention is use of at least one of the compound represented by the general formula [1] and a pharmacologically acceptable salt thereof as an active ingredient for the production of a pharmaceutical composition for treatment or prevention of a disease accompanied by a central nervous system disorder.

EFFECT OF THE INVENTION

The compound of the present invention and the pharmacologically acceptable salt thereof (hereinafter also referred to merely as a compound of the present invention) each has a high affinity to sigma receptors, especially sigma-1 receptors. Also, the compound of the present invention has a neurite outgrowth-promoting activity in central neural cells. Based on these pharmacological actions, the compound of the present invention is applicable as a pharmaceutical composition for treating or preventing a disease accompanied by a central nervous system disorder, particularly as a pharmaceutical composition effective for nerve regeneration or neuronal protection, for example, as a therapeutic agent for a disease accompanied by cranial neuropathy (e.g., Alzheimer's disease, Parkinson's disease, Huntington' s disease, sequelae of cranial neuropathy due to cerebrovascular disorder, dementia, disease accompanied by cerebral neuropathy due to head injury, disease accompanied by motor dysfunction due to spinal cord disorder, and spinal cord damage).

BRIEF EXPLANATION OF THE DRAWINGS

Fig. 1 is a photograph substituted for drawing of the rat cerebral cortex neural cells cultured for 48 hours: (A) no compound added; (B) addition of the compound [2] of the present invention (finial concentration: 1 μM).

Fig. 2 is a graph showing the ratio (%) of cells having a neurite length that is two or more times the length of the cell body diameter to the number of whole cells in the same field of vision, in the rat cerebral cortex neural cells cultured for 48 hours by addition of the compound [2] of the present invention (final concentrations: 0.1 μM, 1 μM, and 10 μM).

MODE FOR CARRYING OUT THE INVENTION

In the compound represented by the above general formula [1], X and Y in the formula each independently represents a hydrogen atom, a halogen atom, a trihalomethyl group, a dihalomethyl group, a monohalomethyl group, a methoxy group, a hydroxyl group, or an alkyl group having 1 to 3 carbon atoms; R1, R2, R3 and R4 each independently represents a hydrogen atom, a hydroxyl group, an alkoxyl group having 1 to 3 carbon atoms (e.g. , methoxy, ethoxy, and propoxy), or an alkyl group having 1 to 3 carbon atoms. When a plurality of R1 and R2 are present, they may be the same or different from each other. R5 represents a hydrogen atom, an alkyl group having 1 to 5 carbon atoms (e.g. , methyl, ethyl, propyl, isopropyl, butyl, isobutyl, sec-butyl, tert-butyl, pentyl, isopentyl, and neopentyl), or an alkylene group which has 2 to 4 carbon atoms and binds to a carbon atom other than a carbon atom bound to a nitrogen atom

on the cyclohexyl ring to form a ring, and n represents an integer of 1 to 5.

In the halogen atom, the trihalomethyl group, the dihalomethyl group, and the monohalomethyl group, the halogen may be fluorine, chlorine, bromine, iodine, or astatine, typically fluorine, chlorine, bromine, or iodine, preferably fluorine or chlorine, and more preferably fluorine.

Examples of the alkylene group mentioned above include those represented by the following general formula (a) (m is an integer of 2 to 4) together with the cyclohexyl ring, for example, those represented by the following formula (b) and those represented by the following formula (c).

(Chemical formula 3)

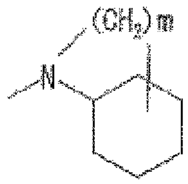

(a)

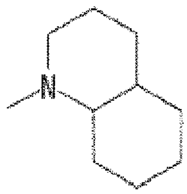

(b)

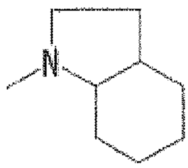

(c)

The compound represented by the above formula [1] is not particularly limited, but includes, for example, a compound wherein X and Y in the formula each independently represents a hydrogen atom, a fluorine atom, a trifluoromethyl group, a difluoromethyl group, a monofluoromethyl group, a methoxy group, a hydroxyl group, or an alkyl group having 1 to 3 carbon atoms, for example, a compound represented by the above formula [1'].

In the above formula [1] and, where applicable, in the formula [1'], X and Y each is independently preferably a hydrogen atom, a fluorine atom, a trifluoromethyl group, a difluoromethyl group, or a methoxy group, and R1, R2, R3 and R4 each is preferably a hydrogen atom. More preferably, X is a hydrogen atom or a methoxy group; Y is a hydrogen atom, a fluorine atom, a trifluoromethyl group, a difluoromethyl group, or a methoxy group; R1, R2, R3 and R4 each is a hydrogen atom; and R5 is an alkyl group having 1 to 3 carbon atoms, or an alkylene group which has 2 to 4 carbon atoms and binds to a carbon atom other than a carbon atom bound to a nitrogen atom on the cyclohexyl ring to form a ring. Further, the substitution positions of X and Y are the 2- to 6-positions, especially the 3- to 5-positions, and further especially the 3- or 4-position of the aromatic ring.

Such compounds include specifically a compound wherein Z represents a sulfur atom, a carbon atom or an oxygen atom, having the groups listed in Tables 1 and 2 shown below, respectively. In the Tables 1 and 2, MeO- represents a methoxy group, the alkyl group represents methyl, ethyl or propyl, and the alkylene represents those represented by the above (b) or those represented by the above (c), respectively.

[Table 1]

| X | Y | R1-R4 | R5 (alkyl, alkylene) | Z |
|---|---|---|---|---|
| H | H | H | alkyl | S |
| H | H | H | alkyl | C |
| H | H | H | alkyl | O |
| H | H | H | alkylene | S |
| H | H | H | alkylene | C |
| H | H | H | alkylene | O |
| H | F | H | alkyl | S |
| H | F | H | alkyl | C |
| H | F | H | alkyl | O |
| H | F | H | alkylene | S |
| H | $F_3C$- | H | alkyl | S |
| H | $F_3C$- | H | alkyl | C |
| H | $F_3C$- | H | alkylene | S |
| H | $F_3C$- | H | alkylene | C |
| H | $F_3C$- | H | alkylene | O |
| H | $F_2HC$- | H | alkyl | S |
| H | $F_2HC$- | H | alkyl | C |
| H | $F_2HC$- | H | alkyl | O |
| H | $F_2HC$- | H | alkylene | C |
| H | $F_2HC$- | H | alkylene | S |
| H | MeO- | H | alkyl | C |
| H | MeO- | H | alkylene | S |
| MeO- | MeO- | H | alkyl | S |
| MeO- | MeO- | H | alkyl | C |
| MeO- | MeO- | H | alkyl | O |
| MeO- | MeO- | H | alkylene | S |

[Table 2]

| X | Y | R1~R4 | R5 (alkyl, alkylene) | Z |
|---|---|---|---|---|
| OH | H | H | alkyl | S |
| OH | OH | H | alkyl | C |
| OH | H | H | alkylene | C |
| F | F | H | alkyl | S |
| F | H | H | alkyl | O |
| H | H | H | alkyl | O |
| $CH_3$- | H | H | alkyl | S |
| $CH_3$- | $CH_3$- | H | alkyl | C |

(continued)

| X | Y | R1~R4 | R5 (alkyl, alkylene) | Z |
|---|---|---|---|---|
| $CH_3$- | F | H | alkyl | S |
| $CH_3$- | $CH_3$- | H | alkyl | O |
| $CH_2F$- | H | H | alkyl | S |
| $CH_2F$- | H | H | alkylene | S |

When such compounds are specifically illustrated, they include, for example, preferably the compounds [2] to [12] mentioned above, and more preferably the compounds [2], [3], [5] and [8].

The pharmacologically acceptable salt is not particularly limited, but includes, for example, salts with inorganic acids (e.g., sulfuric acid, hydrochloric acid, and phosphoric acid) or organic acids (e.g., acetic acid, oxalic acid, lactic acid, tartaric acid, fumaric acid, maleic acid, trifluoroacetic acid, and methanesulfonic acid), and hydrochloride or tartrate is preferred.

As a method for synthesizing the compound of the present invention, for example, the method in the following scheme 1 can be exemplified for the compounds [2] wherein Z is a sulfur atom.

(Chemical formula 4)

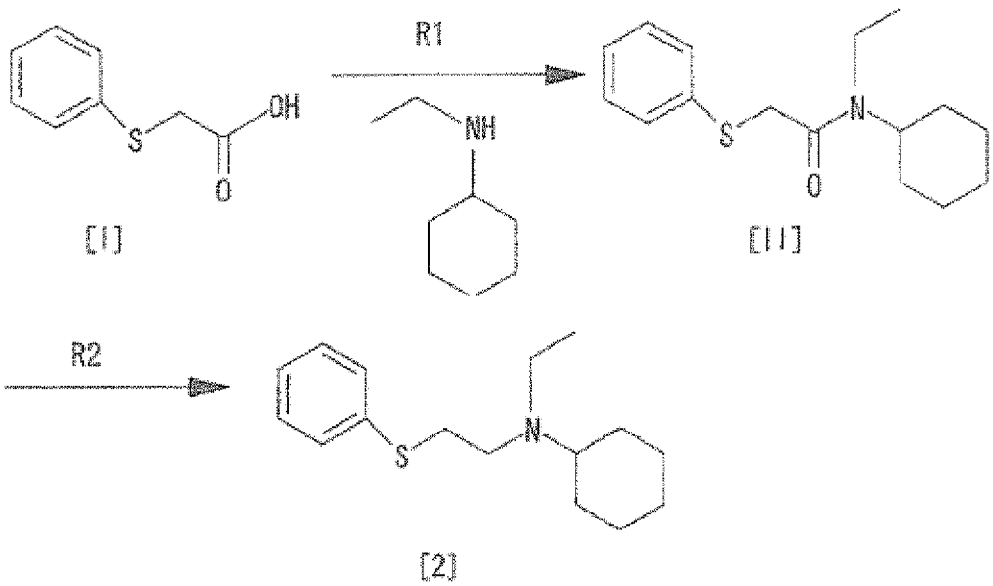


[I] [II] [2]

Scheme 1

This method includes reacting (phenylthio) acetic acid of the formula [I] or its derivative as a starting material with N-ethylcyclohexylamine in the presence of 1-hydroxybenzotriazole and a water-soluble carbodiimide to give a compound of the formula [II] (step R1) and treating this compound with lithium aluminum hydride to yield a compound of the formula [2] (step R2).

The reaction of step R1 is carried out under stirring in a reaction-inert solvent (e.g., anhydrous acetonitrile, anhydrous dichloromethane, anhydrous chloroform, anhydrous benzene, anhydrous toluene, anhydrous N,N-dimethylformamide, and anhydrous dimethyl sulfoxide) at room temperature to 60°C, preferably at room temperature, for 5 to

36 hours, preferably 12 to 24 hours. The reaction of step R2 is carried out under stirring in a reaction-inert solvent (e.g., anhydrous diethyl ether, anhydrous tetrahydrofuran, anhydrous 1,2-dimethoxyethane, and anhydrous diglyme) at -70°C to 60°C, preferably at 0 to 50°C, for 2 to 36 hours, preferably 12 to 24 hours.

The compounds [3] to [7] also can be obtained in the same way, and the compound [4] may be obtained by reacting (3-trifluoromethylphenylthio)acetic acid in place of the compound [I] in the above method; the compound [6] may be obtained by reacting (3,4-dimethoxyphenylthio)acetic acid in place of the compound [I]; and the compound [7] may be obtained by reacting (4-fluorophenylthio)acetic acid in place of the compound [1]. Moreover, in order to obtain the compound [3], decahydroquinoline and the compound of the formula [I] may be reacted in place of the N-ethylcyclohexylamine in the above method. Further, the compound [5] can be obtained by using (3-difluoromethylphenylthio) acetic acid in step R2 for obtaining the compound [4]. However, the compound [5] can also be obtained by separating and purifying the compound [4] and the compound [5] respectively by a silica gel column chromatography after the step of obtaining the compound [4].

In addition, the compound wherein n is 2 and Z is a sulfur atom can be produced by using (phenylthio) propionic acid or its derivative in place of (phenylthio)acetic acid or its derivative. Similarly, the compound wherein n is 3 to 5 can also be produced by using a butanoic acid derivative, a pentanoic acid derivative, a hexanoic acid derivative, etc. Using N-propylcyclohexylamine and the like in place of N-ethylcyclohexylamine, a corresponding compound of the present invention can be produced.

Among the compounds of the present invention, the compound wherein Z is a carbon atom can be produced by using 3-phenylpropionic acid or its derivative in place of (phenylthio) acetic acid or its derivative. For example, as an example of the above compounds [8] to [10] (collectively referred to as compound [A] in the following scheme 2), they can be synthesized by the reaction between N-cyclohexyl-N-ethylamine and a 3-phenylpropionic acid derivative [III] (in the formula, R1 represents H, OH or F), followed by reduction. Scheme 2 is shown below.

(Chemical formula 5)

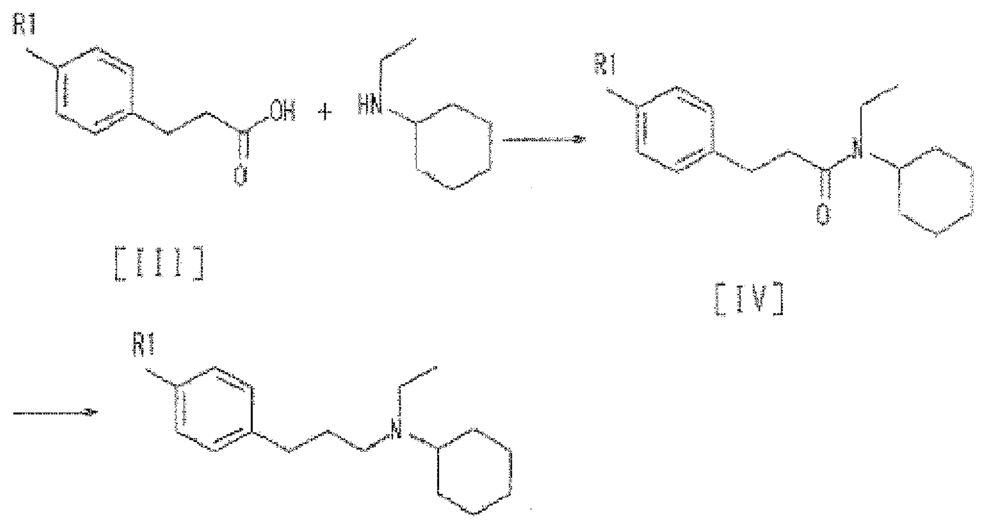

[III]

[IV]

[A]

Scheme 2

In other words, a compound [A] can be obtained by condensing a 3-phenylpropionic acid derivative (formula [III]) with N-cyclohexyl-N-ethylamine to give an amide compound (formula [IV]), which is then reduced.

In addition, a corresponding compound of the present invention (n is 2) can be produced with use of phenylbutanoic acid or its derivative in place of a 3-phenylpropionic acid derivative. Similarity, the compound wherein n is 3, 4,

or 5 can also be produced. Using N-propylcyclohexylamine and the like in place of N-ethylcyclohexylamine, a corresponding compound of the present invention can be produced.

Among the compounds of the present invention, the compound wherein Z is an oxygen atom can be produced by using, for example, 1-(2-haloethoxy) benzene or its derivative in place of (phenylthio)acetic acid or its derivative. For example, as examples of the compounds [11] to [12], they can be synthesized by reacting a 1-(2-bromoethoxy)benzene derivative with N-cyclohexyl-N-ethylamine or cyclohexylamine and optionally reducing the product while referring to the above-mentioned method. Specifically, such synthesis can be performed with reference to the method described in Examples.

In addition, a corresponding compound of the present invention (n is 2) can be produced with use of 1-(2-halopropoxy)benzene or its derivative in place of 1-(2-haloethoxy)benzene or its derivative. Similarly, the compound wherein n is 3, 4, or 5 can also be produced.

The compounds obtained by the above method can be converted into various salts by the usual method.

In addition, there may be optical isomers in the compounds represented by the general formula [1], but all of them should be included in the present invention.

Formulation method of pharmaceutical composition of the present invention

The pharmaceutical composition of the present invention contains as an active ingredient at least one of the compound represented by the general formula [1'] and a pharmacologically acceptable salt thereof, for example, at least one of the compounds [2] to [12] and pharmacologically acceptable salts thereof, especially at least one of the compounds [2], [3], [5], and [8] and pharmacologically acceptable salts thereof, and the dosage form to be used for administration includes, for example, tablets, granules, powders, capsules, syrups, suspensions, and injections. These dosage forms can be prepared using a technique frequently used as a usual formulation method, and, the above active ingredients can be formulated into, for example, oral preparations such as tablets, capsules, and granules, using, where necessary, fillers (e.g., lactose, starch, crystalline cellulose, and vegetable oil), lubricants (e.g., magnesium stearate and talc), binders (e.g., hydroxypropyl cellulose and polyvinylpyrrolidone), disintegrators (e.g., carboxymethyl cellulose calcium), coating agents (e.g., hydroxypropyl cellulose, macrogol, and a silicone resin), gelatin film-forming agents, syrups, suspensions, and injectable purified water solutions.

For example, in the case of capsules, the active ingredient is contained in the dosage unit amount of, for example, 10 mg, 20 mg, 50 mg, or 100 mg i n the form of, for example, a powder, and if necessary, the filler may be incorporated in an amount of, for example, 10 mg, 20 mg, 50 mg, or 100 mg. In the case of tablets, the active ingredient in the dosage unit amount of, for example, 0.1 mg, 1 mg, 2 mg, 5mg, or 10 mg, and the filler in an amount of, for example, 20 to 150 mg may be incorporated. In the case of injections, the active ingredient in an amount of, for example, 10 mg, 50 mg, or 100 mg may be incorporated with distilled water.

Administration method of pharmaceutical composition of the present invention

The administration of the pharmaceutical composition of the present invention may be performed orally (for example, administration in the dosage form of tablets, granules, powders, capsules, syrups, or suspensions) or parenterally (for example, administration with injections). These administration methods and dosage forms can be appropriately selected depending on the symptom, age, therapeutic purpose, etc. of a subject to be administered in need of treatment or prevention.

The therapeutically or preventively effective amount of the active ingredient in the pharmaceutical composition of the present invention is appropriately selected depending on the symptom, age, dosage form, administration route, therapeutic purpose, etc., but, in the case of human adults, it is 0.01 to 100 mg/individual per day and can be increased or decreased depending on the symptom, age, body weight, race, therapeutic purpose, etc. of a patient. The therapeutically or preventively effective amount of the active ingredient is administered once to several times per day. A single unit dose for human adults is administered, for example, preferably in 0.01 to 40 mg/individual, more preferably 0.1 to 10 mg/individual. The therapeutically or preventively effective amount of the active ingredient is an amount effective for nerve regeneration or neuronal protection. Here, it should be understood that the neuronal protection means the treatment or prevention of nerve injury by regeneration/maturation, and preferably promotion thereof, of nerve cells. Thus, optimal concentration should be investigated, for example, in the case of compounds where an effect of promoting regeneration/maturation of neural cells is rather decreased at a high concentration. The amount effective for nerve regeneration or neuronal protection can be known by examining the concentration dependency of the pharmacological effects such as neurite outgrowth-promoting activity. This amount varies depending on the compounds, and the effective amount of the active ingredient can be easily set by those skilled in the art because the concentration dependency of the pharmacological action is proved by an evaluation mentioned below. Further, it is to be mentioned that even compounds with a high affinity to sigma receptors do not always have a neurite outgrowth-promoting activity.

The pharmaceutical composition of the present invention can be applied to the treatment or prevention of a disease accompanied by a central nervous system disorder. The disease accompanied by a central nervous system disorder is not particularly limited, but includes typically Alzheimer's disease, Parkinson's disease, Huntington's disease, sequelae of cranial neuropathy due to cerebrovascular disorder, dementia, disease accompanied by cranial neuropathy due to head injury, and disease accompanied by motor dysfunction due to spinal cord injury. A subject that needs the treatment or prevention is usually a human subject, but application to non-human animals that need the treatment or prevention (mammals, for example, non-human primates such as monkey, gorilla, and chimpanzee; small animals (e.g., dog, cat, and mouse) ; livestock (e.g., cow, horse, and sheep)) is also possible.

Examples

Hereinafter, the present invention will be further described by way of Examples, but it is not intended to be limited thereto.

Example 1

Synthesis of N-(2-phenylthio)ethyl-N-ethylcyclohexylamine hydrochloride

(Chemical formula 6)

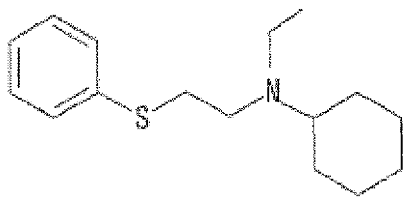

[2]

Under an argon atmosphere, (phenylthio) acetic acid (5.05 g (29.7 mmol)), N-ethylcyclohexylamine (1.40 g (11.0 mmol)), 1-hydroxybenzotriazole (4.87 g (36.0 mol)) and 1-[3-(dimethylamino)propyl]-3-ethylcarbodiimide hydrochloride (6.89 g (35.9 mmol)) were dissolved in anhydrous N,N-dimethylformamide (100 mL), and stirred overnight at room temperature. Subsequently, the reaction solution was added to ice-water (200 mL) , adjusted to pH 4 with 1 mol/mL hydrochloric acid, and extracted with ethyl acetate. The organic layer was washed successively with a saturated aqueous sodium bicarbonate solution and a saturated brine solution, and dried by the addition of anhydrous sodium sulfate. After removal of the sodium sulfate by filtration, the solvent was distilled away under reduced pressure to obtain N-cyclohexyl-N-ethyl- (phenylthio) acetic acid amide as a crude product.

The resulting crude N-cyclohexyl-N-ethyl-(phenylthio)acetic acid amide (6.37 g) was dissolved in anhydrous tetrahydrofuran (300 mL) under an argon atmosphere, and lithium aluminum hydride (8.7 g (229 mmol)) was gradually added to the solution under ice cooling. After returning to room temperature, the solution was warmed to 40°C and stirred overnight. Then, sodium sulfate decahydrate (50 g (155 mmol)) was gradually added thereto under ice cooling, and the mixture was stirred at room temperature for 4 hours. The reaction mixture was dried by the addition of anhydrous sodium sulfate. After removal of the crystal in the vessel by filtration, the solvent was distilled away under reduced pressure to obtain N-cyclohexyl-N-(2-phenylthio)ethyl-N-ethylamine as a crude product. The crude product was dissolved in a mixed solvent of n-hexane/ethyl acetate (3:1), and 4 mol/L HCl/ethyl acetate (4.5 mL (18.0 mmol)) was dropwise added thereto, and then the mixture was stirred at room temperature overnight. The precipitated crystal was collected by filtration, washed with a mixed solvent of n-hexane/ethyl acetate (1: 1), and dried to obtain a crude product of the target N-(2-phenylthio)ethyl-N-ethylcyclohexylamine hydrochloride as a grayish white crystalline powder. The crude product was recrystallized from ethyl acetate to obtain N-(2-phenylthio)ethyl-N-ethylcyclohexylamine hydrochloride as a white crystalline powder (2.68 g, 8.94 mmol, total yield 81.3%).

NMR (DMSO-$d_6$) δ (ppm) :1.00-1.43 (8H, m), 1.57 (1H,m)1.75 (2H,m), 1. 99 (2H,m), 2.95-3.22 (5H,m), 3.50 (2H,m), 7.25 (1H,m), 7.35 (2H,m), 7 .47 (2H,m),10.70 (1H,bs)
IR (KBr, $cm^{-1}$) :2948, 2750-2350, 1460, 1445, 1400, 1040, 1022, 739, 69 2

Example 2

Synthesis of N-(2-phenylthio)ethyldecahydroquinoline hydrochloride

(Chemical formula 7)

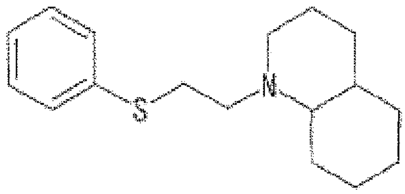

[3]

Under an argon atmosphere, (phenylthio) acetic acid (5.0 g (29.7 mmol)), decahydroquinoline (4.6 g (32.7 mmol)), 1-hydroxybenzotriazolemonohydrate (4.8 g (35.7 mol)) and 1-[3-(dimethylamino)propyl]-3-ethylcarbodiimide hydrochloride (6.8 g (35.7 mmol)) were dissolved in anhydrous N,N-dimethylformamide (70 mL), and the solution was stirred overnight at room temperature. Subsequently, ice-water was added to the reaction mixture, and it was adjusted to pH 4 with a 10% hydrochloric acid, and extracted with ethyl acetate. The organic layer was washed successively with a saturated aqueous sodium bicarbonate solution and a saturated brine solution, and dried by the addition of anhydrous sodium sulfate. After removal of the sodium sulfate by filtration, the solvent was distilled away under reduced pressure to obtain a crude product of N-[(phenylthio)acetyl]decaquinoline as a pale yellow oil.

The resulting crude N-(thiophenoxyacetyl)decaquinoline (5.5 g) was dissolved in anhydrous tetrahydrofuran (200 mL) under an argon atmosphere, and lithium aluminum hydride (7.2 g (190 mmol)) was gradually added to the solution under ice cooling. After returning to room temperature, the mixture was further warmed to 40°C and stirred overnight. Then, sodium sulfate decahydrate (42 g (130 mmol)) was gradually added under ice cooling, and the mixture was stirred at room temperature for 4 hours. The reaction mixture was dried by the addition of anhydrous sodium sulfate. After removal of the crystal in the vessel by filtration, the solvent was evaporated under reduced pressure to obtain N-(2-phenylthio)ethyldecahydroquinoline as a crude product. This crude product was purified by a silica gel column chromatography (n-hexane : ethyl acetate = 1:1). The resulting N-(2-phenylthio)ethyldecahydroquinoline was dissolved in ethyl acetate 10 mL, and 4 mol/L HCl/ethyl acetate (1.0 mL (3.99 mmol)) was dropwise added. Then, after addition of n-hexane 10 mL, the mixture was stirred overnight at room temperature. The precipitated crystal was collected by filtration, and dried to obtain a target N-(2-phenylthio)ethyldecahydroquinoline hydrochloride as a white crystal (0.99 g, 3.17 mmol, total yield 10.7%).

NMR (DMSO-$d_6$) δ (ppm): 0.92-1.38 (5H,m), 1.54-197 (8H,m), 2.86(1H, m), 2.93-3.20 (2H,m), 3.23-3.52 (4H, m), 7.28 (1H,m), 7.34 (2H,m), 7. 42(2H,m), 10.40 (1H,br)
IR (KBr,$cm^{-1}$):2948, 2852, 2500-2250, 1479, 1450, 748, 692

Example 3

Synthesis of

N-[2-(3-trifluoromethyl)phenylthio]ethyl-N-ethylcyclohexyla mine

(Chemical formula 8)

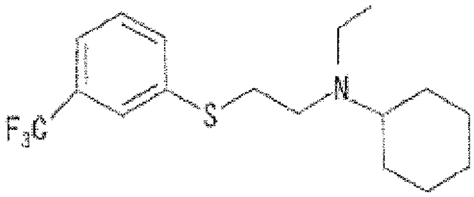

[4]

Under an argon atmosphere, (3-trifluoromethyl-phenylthio)acetic acid (5.3 g (22. 4 mmol)), N-ethylcyclohexylamine (3.1 g (24.7 mmol)), 1-hydroxybenzotriazole monohydrate (3.6 g (26.9 mmol)) and 1-[3-(dimethylamino)propyl]-3-ethylcarbodiimide hydrochloride (5.2 g (26.9 mmol)) were dissolved in anhydrous N,N-dimethylformamide (78 mL), and the solution was stirred overnight at room temperature. Subsequently, the reaction mixture was added to ice-water, adjusted to pH 4 with 10% hydrochloric acid, and extracted with ethyl acetate. The organic layer was washed successively with a saturated aqueous sodium bicarbonate solution and a saturated brine solution, and dried by the addition of anhydrous sodium sulfate. After removal of the sodium sulfate by filtration, the solvent was distilled away under reduced pressure. The residue was purified by a silica gel column chromatography (n-hexane : ethyl acetate = 10 : 1 to 5 : 1) to obtain N-cyclohexyl-N-ethyl-(3-trifluoromethyl-phenylthio)acetic acid amide as a pale yellow oil (4.4 g, 12.7 mmol, yield 56.8%).

The resulting N-cyclohexyl-N-ethyl-(3-trifluoromethylphenylthio)acetic acid amide (4.4 g (12.7 mmol)) was dissolved in anhydrous tetrahydrofuran (200 mL) under an argon atmosphere, and lithium aluminum hydride (4.8 g (127 mmol) was gradually added to the solution under ice cooling. After returning to room temperature, the mixture was stirred overnight. Then, sodium sulfate decahydrate (28 g (86.9 mmol)) was gradually added under ice cooling, and the mixture was stirred at room temperature for 3 hours. Then, the reaction mixture was dried by the addition of anhydrous sodium sulfate. After removal of the crystal in the vessel by filtration, the solvent was distilled away under reduced pressure. The residue was purified by a medium pressure silica gel column chromatography (n-hexane : ethyl acetate = 1 : 1) to obtain a fraction containing a target compound. The solvent was distilled away from the fraction and the residue was dissolved in ethyl acetate. After dropwise addition of 4 mol/L HCl/ethyl acetate (1.0 mL (3.99 mmol)) to the solution, the solvent was distilled away and ethyl acetate was added thereto. The precipitated crystal was collected by filtration and dried to obtain the title compound, N-[2-(3-trifluoromethyl)phenylthio]ethyl-N-ethylcyclohexyla mine (0.33 g, 0.897 mmol, yield 7.0%) as a white crystal.

NMR (DMSO-$d_6$) δ (ppm) :1.02-1.45 (8H,m), 1.77 (2H,m), 1.94 (2H,m), 3. 09-3.38 (5H,m), 3.51 (2H,m), 7.62 (2H,m), 7.74 (2H,m), 9.52 (1H,br) IR (KBr,$cm^{-1}$):2950, 2875, 2700-2400, 1500-1418, 1300, 1780, 1188, 1 162, 1120, 1078, 802, 702

Example 4

Synthesis of

N-[2-(3-difluoromethyl)phenylthio]ethyl-N-ethylcyclohexylam ine

(Chemical formula 9)

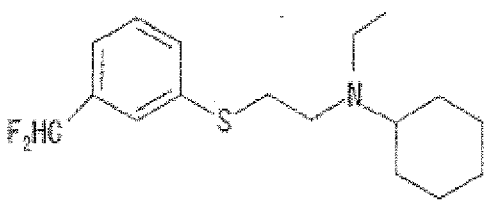

[5]

The target compound (0. 75 g, 2.19 mmol, yield 17.2%) was obtained in the same manner as in Example 3, except that (3-difluoromethyl) thiophenoxyacetic acid was used in place of (3-trifluoromethylphenylthio)acetic acid.

NMR (DMSO-$d_6$) δ (ppm) :0.99-1.49 (8H,m), 1.58 (1H,m), 1.75 (2H,m), 1. 99 (2H,m), 3.06-3.32 (5H,m), 3.55 (2H,m), 7.05 (1H,t,J=54.1Hz), 7.4 6 (1H,m), 7.52 (1H,m), 7.65(2H,m), 10.42 (1H,bs)
IR (KBr, $cm^{-1}$) : 2954, 2.890, 2780-2430, 1474, 1454, 1374, 1238, 1102, 1 088, 1036 ,808, 730, 710

Example 5

Synthesis of

N-[2-(3,4-dimethoxy)phenylthio]ethyl-N-ethylcyclohexylamine L-tartrate

(Chemical formula 10)

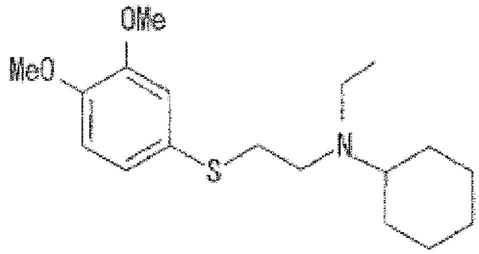

[6]

Under an argon atmosphere,
(3,4-dimethoxyphenylthio)acetic acid (4.8 g (20.9 mmol)), N-ethylcyclohexylamine (2.9 g (23.0 mmol)), 1-hydroxybenzotriazole monohydrate (3.4 g (25.1 mmol)) and 1-[3-(dimethylamino)propyl]-3-ethylcarbodiimide hydrochloride (4.8 g (25.1 mmol)) were dissolved in anhydrous N,N- dimethylformamide (100 mL), and the solution was stirred overnight at room temperature. Subsequently, the reaction mixture was added to ice-water, adjusted to pH 4 with 10% hydrochloric acid, and extracted with ethyl acetate. The organic layer was washed successively with a saturated aqueous sodium bicarbonate solution and a saturated brine solution, and dried by the addition of anhydrous sodium sulfate. After removal of the sodium sulfate by filtration, the solvent was distilled away under reduced pressure to obtain a crude product of N-cyclohexyl-N-ethyl-(3,4-dimethoxyphenylthio) acetic acid amide as a pale yellow oil.

Next, the obtained crude N-cyclohexyl-N-ethyl-(3,4-dimethoxyphenylthio)acetic acid amide was dissolved in anhydrous tetrahydrofuran (350 mL) under an argon atmosphere, and lithium aluminum hydride (7.3 g (193 mmol)) was gradually added to the solution under ice cooling. After returning to room temperature, it was further warmed to 40°C, and then stirred overnight. Then, sodium sulfate decahydrate (43 g (133 mmol)) was gradually added under ice cooling, and the mixture was stirred at room temperature for 4 hours. Then, the reaction mixture was dried by the addition of anhydrous sodium sulfate. After removal of the crystal in the vessel by filtration, the solvent was distilled away under reduced pressure. The residue was dissolved in ethyl acetate (25 mL), and 4 mol/L-HCl/ethyl acetate (2.6mL (10.4 mmol)) was dropwise added. After completion of the dropwise addition, n-hexane (25 mL) was added thereto and the mixture was stirred overnight, followed by distillation away of the solvent under reduced pressure. The residue was dissolved in water (15 mL) and neutralized (pH 10 to 11) with a 10% aqueous sodium hydroxide solution. The solution was extracted with ethyl acetate and the organic layer was dried over anhydrous magnesium sulfate. After removal of the magnesium sulfate by filtration, the solvent was distilled away under reduced pressure to obtain N-(2-(3,4-dimethoxy) phenylthio)ethyl-N-ethylcyclohexylamine (0.76 g, 2.35 mmol). This product was dissolved in ethanol and L-tartaric acid (0.35 g (2.35 mmol)) was added to the solution, followed by stirring. After the dissolution of L-tartaric acid, the solvent was distilled away and diisopropyl ether was added to the residue. Then, the solvent was removed and the residue was dried to obtain a target N-(2-(3,4-dimethoxy)phenylthio)ethyl-N-ethylcyclohexylamine L-tartrate as a pale yellow crystal (0.7 g, 1.48 mmol, total yield 7.07%).

NMR(DMSO-$d_6$) δ (ppm) : 0.94-1.24(8H,m), 1.55(1H,m), 1.71(4H,m),2. 66(3H,m), 2.73(2H,m), 2.98(2H,m), 3.40(br), 3.73(3H,s), 3.76(3H, s), 4.14(2H,2), 6.93(2H,m), 6.94(1H,m)
IR(KBr,$cm^{-1}$):3650-3180, 3025-2820, 1780-1700, 1658-1560, 1505, 1 255, 1230, 1010, 810, 766, 682, 608

Example 6

Synthesis of

N-[2-(4-fluorophenylthio)ethyl]-N-ethylcyclohexylamine

(Chemical formula 11)

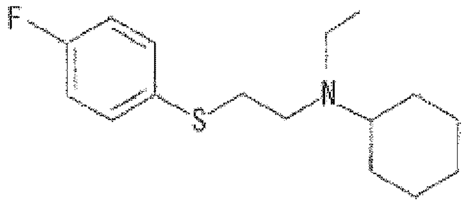

[7]

Under an argon atmosphere, (4-fluorophenylthio)acetic acid (4.87 g (26.9 mmol)), N-ethylcyclohexylamine (3.5g (29.5 mmol)), 1-hydroxybenzotriazolemonohydrate (4.1g (32.2 mmol)) and 1-[3-(dimethylamino)propyl]-3-ethylcarbodiimide hydrochloride (5.8 g (32.2 mmol)) were dissolved in anhydrous N,N-dimethylformamide (100 mL), and stirred overnight at room temperature. Subsequently, the reaction mixture was added to ice-water, adjusted to pH 4 with 10% hydrochloric acid, and extracted with ethyl acetate. The organic layer was washed successively with a saturated aqueous sodium bicarbonate solution and a saturated brine solution, and dried by the addition of anhydrous sodium sulfate. After removal of the sodium sulfate by filtration, the solvent was distilled away under reduced pressure to obtain a crude product of N-cyclohexyl-N-ethyl-(4-fluorophenylthio)acetic acid amide as a pale yellow oil.

Next, the resulting crude N-cyclohexyl-N-ethyl-(4-fluorophenylthio)acetic acid amide was dissolved in anhydrous tetrahydrofuran (350 mL) under an argon atmosphere, and lithium aluminum hydride (9.0 g (237 mmol)) was gradually added to the solution under ice cooling. After returning to room temperature, it was further warmed to 40°C, and stirred overnight. Then, sodium sulfate decahydrate (52 g (161 mmol)) was gradually added under ice cooling, and the mixture was stirred at room temperature for 4 hours. The reaction mixture was then dried by the addition of anhydrous sodium sulfate. After removal of the crystal in the vessel by filtration, the solvent was distilled away under reduced pressure. The residue was purified by a silica gel column chromatography (n-hexane : ethyl acetate = 1 : 1) to obtain N-[2-(4-fluorophenylthio)ethyl]-N-ethyl-cyclohexylamine, which was dissolved in ethyl acetate (37 mL), followed by dropwise addition of 4 mol/L-HCl/ethyl acetate (4.2 mL (16.8 mmol)). After completion of the dropwise addition, n-hexane (37 mL) was added thereto and the mixture was stirred overnight. The precipitated crystal was collected by filtration and purified by a medium pressure reversed-phase silica gel column chromatography (acetonitrile : 1% hydrochloric acid = 3 : 7). Then, the product was freeze-dried, crystallized from a mixed solvent of ethyl acetate-ether and dried to obtain the target N-(2-(4-fluorophenylthio)ethyl]-N-ethylcyclohexylamine hydrochloride as a white crystal (0.96g, 3.02 mmol, total yield 11.2%).

NMR(DMSO-$d_6$)δ(ppm):0.98-1.46(8H,m), 1.58(1H,m), 1.76(2H,m),1. 98(2H,m), 3.00-3.30(5H,m), 3.42(2H,m), 7.25(2H,m), 7.54(2H,m), 1 0.20(1H,bs)
IR(KBr,$cm^{-1}$):2940, 2875, 2600-2345, 1494, 1458, 1218, 835, 620

Example 7

Synthesis of N-(3-phenylpropyl)-N-ethylcyclohexylamine

(Chemical formula 12)

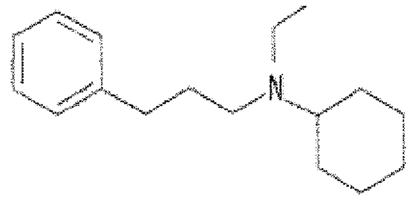

[8]

Under an argon atmosphere, N-cyclohexyl-N-ethylamine (4.65 g (36.6 mmol)), 3-phenylpropionic acid (5.05 g (36.6 mmol)), 1-hydroxybenzotriazole monohydrate (5.44 g (40.3 mmol)) and 1-ethyl-3-(3-(dimethylaminopropyl)carbodiimide hydrochloride (7.66 g (40.0 mmol)) were dissolved in anhydrous N,N- dimethylformamide (100 mL), and stirred overnight at room temperature. The reaction mixture was added to ice-water (200 mL), adjusted to pH with 1N hydrochloric acid, and extracted with ethyl acetate. The organic layer was washed successively with a saturated aqueous sodium bicarbonate solution and a saturated brine solution, dried over anhydrous sodium sulfate, and concentrated under reduced pressure to obtain a crude N-cyclohexyl-N-ethyl-(3-phenylpropionic acid)amide as a pale yellow oil.

1H-NMR($CDCl_3$)δ(ppm);1.10 and 1.13(total 3H, each t, each J=9.5Hz, 9.3Hz), 1.22-1.37 and 1.57-1.85(10H,m), 2.56-2.64(2H,m), 2.88-3.01(2H,m), 3.14 and 3.28(total2H, each q, each J=9.5Hz, 9.3Hz), 3.31-3.49 and 4.31-4.41(total1H, each m), 7.19-7.32(5H,m)

The crude N-cyclohexyl-N-ethyl-(3-phenylpropionic acid)amide (4.49g) was dissolved in anhydrous tetrahydrofuran (250 mL) under an argon atmosphere, and lithium aluminum hydride (6.6 g (174 mmol)) was gradually added to the solution under ice cooling, followed by stirring overnight at 40°C. The reaction mixture was cooled with ice and sodium sulfate decahydrate (40 g (124 mmol)) was gradually added under ice cooling. After returning to room temperature, it was stirred for further 4 hours. The reaction mixture was dried over anhydrous sodium sulfate, and concentrated under reduced pressure. The resulting oil was purified by a silica gel column chromatography to obtain the target compound as a colorless oil (1.64 g, 6.68 mmol, total yield 19.9%).

1H-NMR($CDCl_3$)δ(ppm);1.00(3H, t, each J=7.2Hz), 1.04-1.25(5H,m), 1.59-1.62(1H,m), 1.7-1.79(6H,m),2. 43-2.63(7H,m), 7.15-7.29(5H,m)

IR(ATR, $cm^{-1}$); 3085, 3062, 3026, 2928, 2853, 2807, 1604, 1496, 1451, 1 377, 1345, 1301, 1261, 1195, 1174, 1120, 1077, 1031, 890, 745, 698, 497

Example 8

Synthesis of

N-[3-(4-methoxyphenyl)propyl]-N-ethyl-cyclohexylamine

(Chemical formula 13)

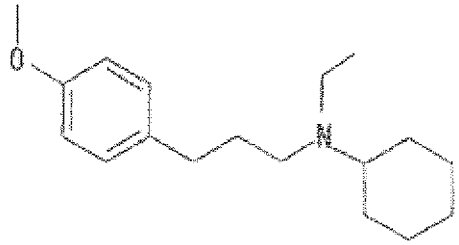

[9]

Under an argon atmosphere, N-cyclohexyl-N-ethylamine (3.84 g (30.2 mmol)), 3-(4-methoxyphenyl)propionic acid (4.84 g (26.9 mmol)), 1-hydroxybenzotriazole monohydrate (4.30 g (31.8 mmol)) and

1-ethyl-3-(3-(dimethylaminopropyl)carbodiimide hydrochloride (6.20g (32.3 mmol)) were dissolved in anhydrous N,N-dimethylformamide (100 mL), and stirred overnight at room temperature. The reaction mixture was added to ice-water (200 mL)), adjusted to pH 4 with 1N hydrochloric acid, and extracted with ethyl acetate. The organic layer was washed successively with a saturated aqueous sodium bicarbonate solution and a saturated brine solution, dried over anhydrous sodium sulfate, and concentrated under reduced pressure to obtain a crude N-cyclohexyl-N-ethyl-[3-(4-methoxyphenyl) propionic acid]amide (6.84 g) as a pale yellow oil.

1H-NMR($CDCl_3$)δ(ppm); 1.08-1.14(3H,m), 1.21-1.85(10H,m), 2.54-2 .60(2H,m), 2.88-2.95(2H,m), 3.14 and 3.27(total2H, each q, each J=7.1Hz, 7.1Hz), 3.41-3.49 and 4.31-4.41(total1H, each m), 3.83(3H,s)6.81-6.85(2H,m), 7.13-7.16(2H,m)

The crude N-cyclohexyl-N-ethyl-[3-(4-methoxyphenyl)propionic acid]amide (6.80 g) was dissolved in anhydrous tetrahydrofuran (300 mL) under an argon atmosphere, and lithium aluminum hydride (8.8 g (232 mmol)) was gradually added to the solution under ice-water cooling, followed by stirring overnight at 40°C. The reaction mixture was cooled with ice and sodium sulfate decahydrate (53 g (164 mmol)) was gradually added thereto. After returning to room temperature, the mixture was further stirred for 4 hours. The reaction mixture was dried over anhydrous sodium sulfate, and concentrated under reduced pressure. The resulting oil was purified by a silica gel column chromatography to obtain the target compound as a pale yellow oil (3.33 g, 12.1 mmol, total yield 45.0%).

1H-NMR($CDCl_3$) δ (ppm) ; 1.00(3H,t, each J=7.1Hz), 1.04-1.23(5H,m), 1.58-1.65(1H,m), 1.69-1.81(6H,m), 2. 43-2.59(7H,m), 3.79(3H,m), 6.81-6.83(2H,m), 7.09-7.12(2H,m) IR(ATR,$cm^{-1}$); 3060, 3030, 2928, 2853, 2833, 2805, 1612, 1584, 1512,1 465, 1450, 1375, 1345, 1300, 1246, 1175, 1121, 1039, 890, 823, 806, 699 ,558, 520

Example 9

Synthesis of N-[3-(4-fluorophenyl)propyl]-N-ethylcyclohexylamine

(Chemical formula 14)

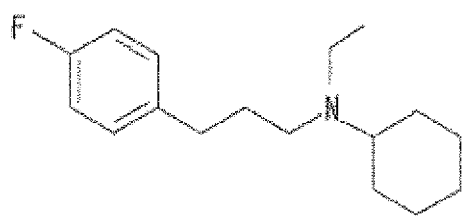

[10]

Under an argon atmosphere, N-cyclohexyl-N-ethylamine (3.90 g (30.7 mmol)), 3-(4-fluorophenyl)propionic acid (4.58 g (27.2 mmol)), 1-hydroxybenzotriazole monohydrate (4.40 g (32.6 mmol)) and 1-ethyl-3-(3-dimethylaminopropyl)carbodiimide hydrochloride (6.30 g (32.9 mmol)) were dissolved in anhydrous N,N-dimethylformamide (100 mL), and stirred overnight at room temperature. The reaction mixture was added to ice-water (200 mL), adjusted to pH 4 with 1N hydrochloric acid, and extracted with ethyl acetate. The organic layer was washed successively with a saturated aqueous sodium bicarbonate solution and a saturated brine solution, dried over anhydrous sodium sulfate, and concentrated under reduced pressure to obtain a crude N-cyclohexyl-N-ethyl-[3-(4-fluorophenyl) propionic acid]amide (5.30 g) as a pale yellow oil.

1H-NMR($CDCl_3$)δ(ppm); 1.06-1.13(4H,m), 1.22-1.28(1H,m), 1.33-1. 49(3H,m), 1.59-1.66 (3h,m), 1.79-1.82 (2H,m), 2.35-2.61(2H,m), 2. 93-3.00(2H,m), 3.14 and 3.27(total2H, each q, each J=7.1Hz, 7.1Hz), 3.41-3.49 and 4.31-4.41(total1H, each m), 6.94-6.99(2H,m), 7.16-7.20(2H,m)

The crude N-cyclohexyl-N-ethyl-[3-(4-fluorophenyl)propionic acid] amide (5.00g) was dissolved in anhydrous tetrahydrofuran (300mL) under an argon atmosphere, and lithium aluminum hydride (6.8 g (179 mmol)) was gradually added to the solution under ice-water cooling, followed by stirring overnight at 40°C. The reaction mixture was cooled with ice and

sodium sulfate decahydrate (40g (124 mmol)) was gradually added thereto. After returning to room temperature, the mixture was further stirred for 4 hours. The reaction mixture was dried over anhydrous sodium sulfate, and then concentrated under reduced pressure. The resulting oil was purified by a silica gel column chromatography to obtain the target compound as a colorless oil (4.02 g, 15.3 mmol, total yield 56.30).

1H-NMR($CDCl_3$)δ(ppm); 0.98-1.25(8H,m), 1.58-1.65(1H,m), 1.68-1. 78(6H,m), 2.44-2.60(7H,m), 6.93-6.97(2H, m), 7.11-7.15 (2H,m) IR(ATR,$cm^{-1}$); 3038, 2964, 2927, 2854, 2807, 1601 1509, 1449, 1375, 1 345, 1261,1222, 1197, 1174, 1156, 1121, 1091, 1016, 890, 846, 822, 700, , 546, 418

Example 10

Synthesis of N-ethyl-N-[2-(4-fluorophenoxy)ethyl]cyclohexylamine

(Chemical formula 15)

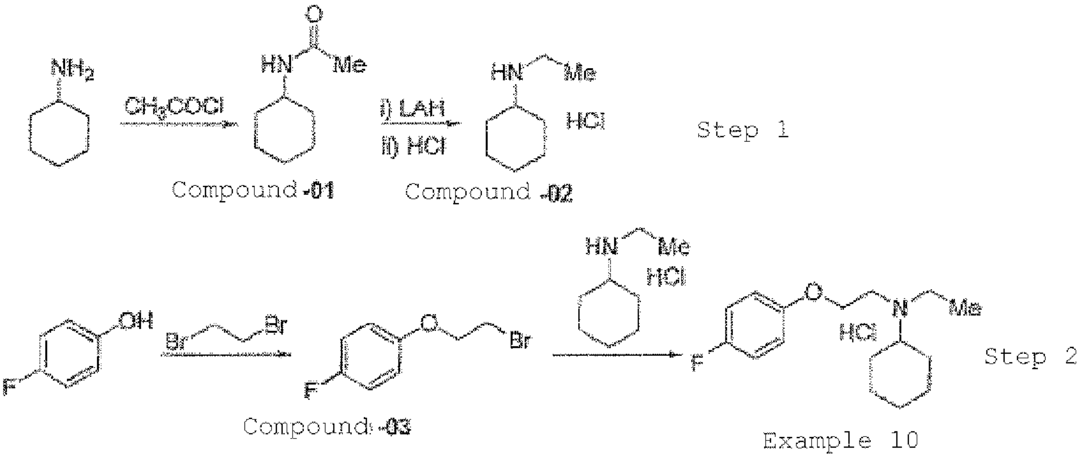


Step 1: Cyclohexylamine (50.0 g (0.5 mol)) was dissolved in dichloromethane (500 mL), and triethylamine (77g (0.75 mol)) was added. The solution was cooled to -30°C, and acetyl chloride (60 g (0.75 mol)) was then dropwise added while keeping the temperature at -30°C. After completion of the dropwise addition, stirring was carried out at room temperature for 2 hours. The insoluble fraction was then removed by filtration and the filtrate was concentrated under reduced pressure to give a crystal, which was recrystallized from hexane/ethyl acetate (2 : 1) to obtain N-cyclohexylacetamide (compound-01) (49.6 g, 0.35 mol, yield 69%).

Lithium aluminum hydride (15.0 g (0.40 mol)) was suspended in tetrahydrofuran (300 mL) under ice-water cooling, and N-cyclohexylacetamide (27.8 g (0.20 mol)) was gradually added. The mixture was stirred for 12 hours under heating at reflux. Then, the temperature was returned to room temperature, and water (3.6 mL) and a 10% aqueous sodium hydroxide solution (27 mL) were successively added to the reaction mixture under ice-water cooling. After removal of the insoluble fraction by filtration, the organic layer was dried over anhydrous sodium sulfate, and the solvent was distilled away under reduced pressure. Hydrogen chloride gas was passed into the residue to form a crystal, which was collected by filtration to obtain N-ethyl-N-cyclohexylamine (compound-02) hydrochloride (14.0g, 9 mmol, yield 56%).

Step 2: A 22.5% aqueous sodium hydroxide solution (55 mL) was added to a mixture of 1,2-dibromoethane (112 g (0.60 mol)) and 4-fluorophenol (16.8 g (0.15 mol)), and the mixture was stirred 5 hours under heating at reflux. Sodium hydroxide (3.0 g (75 mmol)) was further added thereto and the mixture was stirred for 5 hours under heating at reflux. After completion of the reaction, the temperature was returned to room temperature, and the reaction solution was extracted three times with dichloromethane (100 mL). The whole organic layer was dried over $Na_2SO_4$ and the solvent was distilled away under reduced pressure. The residue was purified by a silica gel chromatography (petroleum ether: ethyl acetate = 10: 1) to obtain 1-(2-bromoethoxy)-4-fluorobenzene (compound-03) (29.7 g, 0.136 mol, yield 90%).

1-(2-Bromoethoxy)-4-fluorobenzene (25.5 g (0.116 mol)) and N-ethylcyclohexylamine hydrochloride (28.5 g (0.174 mol)) were dissolved in ethanol (400 mL), and then potassium carbonate (48.7 g (0.35 mol)) and sodium iodide (44.1 g (0.232 mol)) were added to the solution. The mixture was stirred for 48 hours under heating at reflux, and the reaction mixture was returned to room temperature. The insoluble fraction was removed by filtration and the solvent was

distilled away under reduced pressure. The residue was purified by a silica gel chromatography (petroleum ether: ethyl acetate = 6 : 1) and hydrogen chloride gas was passed into the resulting oil to precipitate a crystal, which was collected by filtration to obtain the target N-ethyl-N-[2-(4-fluorophenoxy)ethyl]cyclohexylamine hydrochloride (5.3 g, 17.6 mmol, yield 24%).

Example 11

Synthesis of N-ethyl-N-(2-phenoxy)ethylcyclohexylamine

(Chemical formula 16)

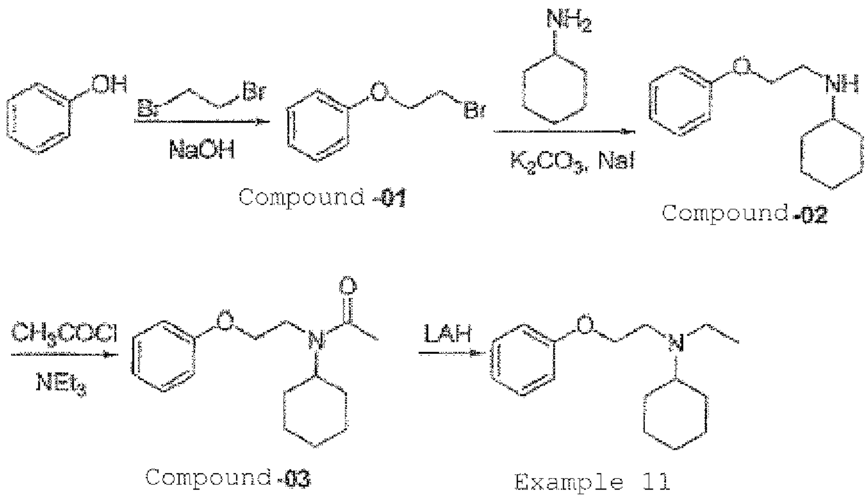


Compound-01

Compound-02

Compound-03

Example 11

A 22.5% aqueous sodium hydroxide solution (135 mL) was added to a mixture of phenol (30.0 g (0.32 mol)) and 1,2-dibromoethane (237 g (1.28 mol)), and the mixture was stirred 5 hours under heating at reflux. The temperature was returned to room temperature, and the mixture was then extracted three times with dichloromethane (100 mL). The whose organic layer was dried over anhydrous sodium sulfate and the solvent was distilled away under reduced pressure. The residue was purified by a silica gel chromatography (petroleum ether : ethyl acetate = 10 : 1) to obtain (2-bromoethoxy) benzene (compound-01) (43.0 g, 0.215 mol, yield 67%).

2-(Bromoethoxy)benzene (43.0 g (0.215 mol)) and cyclohexylamine (53.0 g (0.54 mol)) were dissolved in ethanol (400 mL), and then potassium carbonate (100.0 g (0.72 mol)) and sodium iodide (30.0 g (0.16 mol)) were added thereto. The mixture was stirred for 12 hours under heating at reflux. After the reaction mixture was returned to room temperature, the insoluble fraction was removed by filtration and the solvent was distilled away under reduced pressure. The residue was purified by a silica gel chromatography (petroleum ether : ethyl acetate = 10 : 1) to obtain N-(2-phenoxyethyl) cyclohexylamine (compound-02) (32.0 g, 0.15 mol, yield 68%).

N-(2-Phenoxyethyl)cyclohexylamine (11.0 g (50.0 mmol)) and triethylamine (7.6 g (75 mmol)) were dissolved in dichloromethane (100 mL), cooled to -30°C, and acetyl chloride (6 g (75 mmol)) was slowly added at -30°C, followed by stirring for 2 hours. The insoluble fraction in the reaction mixture was removed by filtration and the solvent was distilled away under reduced pressure. The residue was purified by a silica gel chromatography (petroleum ether: ethyl acetate = 5 : 1) to obtain N-cyclohexyl-N-(2-phenoxyethyl)acetamide (compound03) (12.1 g, 46 mmol, yield 92.4%).

Lithium aluminum hydride (3.5 g (82.0 mmol)) was suspended in tetrahydrofuran (150 mL) under ice-water cooling, and N-cyclohexyl-N-(2-phenoxyethyl)acetamide (11.0 g (42.0 mmol)) was gradually added, followed by stirring for 12 hours at room temperature. Then, the temperature was returned to room temperature, and water (1.6 mL) and a 10% aqueous sodium hydroxide solution (3.5 mL) were added to the reaction mixture under ice-water cooling, after which time the insoluble fraction was removed by filtration. The organic layer was dried over anhydrous sodium sulfate, and the solvent was distilled away under reduced pressure to obtain the target N-ethyl-N-(2-phenoxy)ethylcyclohexylamine (5.8 g, 24.0 mmol, yield 56%).

Pharmacological test of active ingredient in pharmaceutical composition of the present invention

1. Evaluation of affinity to sigma receptors

A membrane preparation as a sigma receptor obtained from the whole brain of guinea pigs was used as an assay system; [$^3$H] (+) -pentazocine was used as a radioactive ligand; (+) -pentazocine was used as a positive substance; and a 50 mM Tris-hydrochloride buffer (pH 7.4) was used as a buffer. In addition, compounds [2] to [12] were used as the test substances. A sample was prepared by weighing each of the test substances, a reference compound or a positive substance and adjusting the final concentrations of the test substances, reference compound (SA4503), and positive substance (haloperidol) as shown below with a buffer.
Concentrations (mol/L): $1 \times 10^{-6}$, $1 \times 10^{-7}$, $1 \times 10^{-8}$, $1 \times 10^{-9}$, $1 \times 10^{-10}$, and $1 \times 10^{-11}$
In this regard, the reference compound for comparative study was also measured.
The inhibition rate and $IC_{50}$ of the test substance, reference compound, and positive substance were determined from a receptor binding experiment by the following method. The following measurement of radioactivity was performed according to a usual method after the binding reaction had been carried out at 25°C for 60 minutes.
The inhibition rate was determined by the equation of "100 - binding rate". The binding rate is represented by the following equation:

$$[(B - N)/(B_0 - N)] \times 100(\%)$$

wherein B is a binding radioactivity to a membrane preparation in the presence of a test substance, a reference compound or a positive substance and a radioactive ligand; $B_0$ is a binding radioactivity (average value) to a membrane preparation in the absence of a test substance, a reference compound, and a positive substance and only in the presence of a radioactive ligand added; and N is a non-specific binding radioactivity (average value).
The $TC_{50}$ of the test substance was calculated from a regression equation to the dose-response curve. That is to say, the ratio y= (B - N)/($B_0$ - N) wherein a value of (B - N) is a specific binding radioactivity in the presence of a test substance, and a value of ($B_0$ - N) is a specific binding radioactivity in the absence of a test substance was first subjected to logit conversion and applied to a logit-log model plotted against the common logarithm of the test substance concentration to create a dose-response curve. In the same way, the $IC_{50}$ values of the reference compound and the positive substance were determined. The regression equation to the dose-response curve used is as follows:

$$Y = aX + b$$

wherein Y = logity = ln(y/(1 - y), X = log x, and a and b are each a constant, provided that x is a solute concentration of a test substance. The results of compounds [3] to [7] are shown in Table 3.
[Table 3]

| Compound | Inhibition rate (%) [Compound concentration mol/L] | | | | | | $IC_{50}$ (nmol/L) |
|---|---|---|---|---|---|---|---|
| | $1\times10^{-11}$ | $1\times10^{-10}$ | $1\times10^{-9}$ | $1\times10^{-8}$ | $1\times10^{-7}$ | $1\times10^{-6}$ | |
| Compound [3] | 2.84 | 6.31 | 46.83 | 90.41 | 100.00 | 99.93 | 1.20 |
| Compound [4] | 10.44 | 23.54 | 95.17 | 99.68 | 100.00 | 100.00 | 0.189 |
| Compound [5] | 1.25 | 20.83 | 92.83 | 98.58 | 99.21 | 97.47 | 0.222 |
| Compound [6] | 1.75 | 19.90 | 86.56 | 98.07 | 99.13 | 99.89 | 0.268 |
| Compound [7] | 0.00 | 3.43 | 44.49 | 90.02 | 96.27 | 97.31 | 1.23 |
| Reference compound | 0.68 | 7.84 | 23.72 | 75.87 | 94.95 | 99.73 | 2.80 |
| Positive substance | 2.05 | 5.16 | 11.41 | 58.79 | 92.11 | 98.77 | 6.22 |

Then, for each concentration of the positive substances, the reaction rate was measured while changing the

concentration of the test substance in a range including the $IC_{50}$ to create the Dixon plot, and the Ki value was calculated from the test substance concentration giving an intersection of these straight lines. The results were shown in Table 4.

2. Evaluation of neurite outgrowth-promoting activity in central neural cells

The neurite outgrowth-promoting effects of the rat cerebral cortex neural cells on test substances were evaluated by the following method. The cerebral cortex neural cells were isolated from Wistar rats on the 18th day of pregnancy according to the method of Springer Neuroscience Lab Manual. That is to say, under ether anesthesia, a fetal rat was removed from the uterus of a pregnant rat on day 18, and transferred into a petri dish containing HBSS (manufactured by Gibco, Inc.) on ice. The cerebral cortex was cut out under a stereoscopic microscope. The cerebral cortex that had been cut out was dispersed with a neural cells dispersion solution (manufactured by Sumitomo Bakelite Co., Ltd.) to prepare rat cerebral cortex cells.

The resulting cerebral cortex cells were subjected to cell culture. Neurobasal medium (manufactured by Gibco, Inc.) supplemented with B27 Supplement (manufactured by Gibco, Inc.; Fin. 2% v/v), L-glutamine (manufactured by IBCO company; Fin. 0.5 mM) and GlutaMax I (manufactured by Gibco, Inc.; Fin. 0.5 mM) was used as the culture medium. The culture conditions were as follows: 5% $CO_2$, 95% air, 100% humidity, and 37°C. The cells were inoculated to polylysine-coated 8-well slides (manufactured by IWAKI company) so as to have about $2 \times 10^4$ cells/well. Compound [2], compound [3], compound [4], compound [5], compound [6], compound [7], compound [8], compound [9], compound [10], compound [11], and compound [12] (final concentrations: 0.3 μM and 1.0 μM) were added as respective test substances to the culture solution at about 3 hours after the cell inoculation, and the culture solution with no additives was used as a control.

At 48 hours after the addition of the compounds, the cells were fixed by treatment with 4% paraformaldehyde (manufactured by Wako Pure Chemical Industries) at room temperature for about 30 minutes, and incubated with an anti-α-Tublin antibody (Anti-α-Tubulin Alexa Fluor 488 conjugate) recognizing a microtubule protein, at room temperature for one hour. After washing, the nucleus of the whole cell was stained with a nucleic acid-staining agent DAPI. The cells were observed under a fluorescence microscope at a magnification of 20 times. The stained cell image and the nuclear straining image in the same field of vision were imported into a computer as the image. Using an image analysis software (MethaMorph (registered trademark), Molecular Devises), the image imported into the computer was used to measure the "longest neurite length" that was the length of the longest neurite of individual cell in each field of vision. A cell having a length of the "longest neurite length" of 60 μm or more was defined as a "neurite forming cell" and the neurite forming activity was evaluated by the ratio of the "neurite forming cell" occupied in the number of the cells in the same field of vision. In other words, evaluation was performed by determining the rate of the neurite forming cells using the following equation:

Rate of neurite forming cells = [Number of neurite forming cells /Number of total cells in the same field of vision] × 100

The results were shown in Table 4. Each numerical value represents mean ± standard error. Further, the results of compound [2] were shown in Figs. 1 and 2.

It was observed from the results of Fig. 1 that the ratio of cells extending dendrites in the rat cerebral cortex neural cells cultured for 48 hours after addition of the compound [2] (final concentration: 1 μM) of the present invention was obviously higher than that of the case of no additives.

It was found from the results of Fig. 2 that the rat cerebral cortex neural cells cultured for 48 hours after respective addition of the compound [2] (final concentrations: 0.1 μM, 1 μM, 10 μM) of the present invention significantly increased the ratio of cells having neurites having the length two or more times the cell body diameter, and thus it became clear that the compound showed a neurite outgrowth-promoting activity.

[Table 4]

| | Sigma receptor binding activity | Neurite outgrowth-promoting activity (%) | |
|---|---|---|---|
| | Ki (nM) | 0.3 μM | 1.0 μM |
| Preference compound | 7.4 | 44.6 ± 2.2 | 58.4 ± 1.8 |
| Compound [2] | 1.0 | 37.4 ± 3.5 | 50.6 ± 1.4 |

(continued)

| | Sigma receptor binding activity | Neurite outgrowth-promoting activity (%) | |
|---|---|---|---|
| | Ki (nM) | 0.3 μM | 1.0 μM |
| Compound [3] | 1.6 | 32.8 ± 5.3 | 52.6 ± 1.2 |
| Compound [4] | 0.8 | 21.4 ± 2.2 | 39.4 ± 2.5 |
| Compound [5] | -* | 41.6 ± 3.3 | 50.8 ± 2.8 |
| Compound [6] | 3.7 | -* | 40.3 ± 3.4 |
| Compound [7] | 0.8 | 30.2 ± 2.5 | 53.3 ± 1.4 |
| Compound [8] | 8.7 | 59.9 ± 1.8 | 61.7 ± 2.0 |
| Compound [9] | 10.5 | 31.4 ± 5.8 | 59.6 ± 1.0 |
| Compound [10] | 8.5 | 26.8 ± 1.1 | 57.0 ± 3.0 |
| Compound [11] | 2.4 | 93.5 ± 2.4 | 50.7 ± 2.2 |
| Compound [12] | 6.6 | 44.2 ± 1.4 | 32.2 ± 1.0 |
| Control | - | 18.0 ± 1.6 | |
| * Data loss | | | |

From the results of Table 4, it was clear that all these compounds of the present invention inhibited the specific binding of the radioactive ligand to a sigma receptor at a very low concentration compared to the reference compound which is a sigma receptor ligand.

In addition, as for the rat cerebral cortex neural cells cultured for 48 hours after addition of each of these compounds of the present invention, it became clear that the ratio of cells each having a neurite length of 60 μm or more was significantly increased, and such compounds were found to have a neurite outgrowth-promoting activity.

Examples of general preparations such as oral preparations and injections of the pharmaceutical composition according to the present invention are shown below. Preparation example 1

Tablet

| | |
|---|---|
| Compound [2] of the present invention | 1 mg |
| Lactose | 55 mg |
| Corn starch | 30 mg |
| Carboxymethyl cellulose calcium | 10 mg |
| Hydroxypropyl cellulose | 3 mg |
| Magnesium stearate | 1 mg |
| Total | 100 mg |

A coating was applied to a tablet having the above formulation using a coating agent (2 mg) (for example, a common coating agent such as hydroxymethyl cellulose, macrogol, and a silicone resin), thereby making it possible to obtain the target tablet with coating (the same as in the tablet with the following formulation). In addition, the target tablet can be obtained by using other compound of the present invention or changing the amount of additives appropriately.

Preparation example 2

Capsule

| | |
|---|---|
| Compound [2] of the present invention | 5 mg |
| Lactose | 145 mg |
| Total | 150 mg |

The target capsule can be obtained by using other compound of the present invention or changing the amount of additives appropriately.

Preparation example 3

Injection

| | |
|---|---|
| Compound [2] of the present invention | 10 mg |
| Sodium chloride | 0.9 mg |
| Sodium hydroxide (or hydrochloric acid) | q.s. |
| Sterile purified water | q.s. |
| Total | 10 ml |

The target injection can be obtained by using other compound of the present invention or changing the amount of additives appropriately.

## Claims

1. A compound represented by the following general formula [1] or a pharmacologically acceptable salt thereof:

(Chemical formula 1)

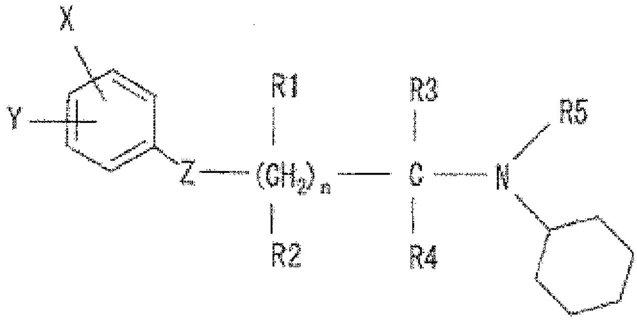

[1]

wherein X and Y each independently represents a hydrogen atoms, a halogen atom, a trihalomethyl group, a dihalomethyl group, a monohalomethyl group, a methoxy group, a hydroxyl group, or an alkyl group having 1 to 3 carbon atoms; R1, R2, R3 and R4 each independently represents a hydrogen atom, a hydroxyl group, an alkoxyl group having 1 to 3 carbon atoms, or an alkyl group having 1 to 3 carbon atoms, provided that when a plurality of R1 and R2 are present, they may be the same or different from each other; R5 represents a hydrogen atom, an alkyl group having 1 to 5 carbon atoms, or an alkylene group which has 2 to 4 carbon atoms and bands to a carbon atom other than a carbon atom bound to a nitrogen atom on the cyclohexyl ring to form a ring; n represents an integer of 1 to 5; and Z represents a sulfur atom, a carbon atom, or an oxygen atom.

2. The compound according to claim 1, wherein X and Y in the general formula [1] each independently represents a hydrogen atom, a fluorine atom, a trifluoromethyl group, a difluoromethyl group, a monofluoromethyl group, a methoxy group, a hydroxyl group, or an alkyl group having 1 to 3 carbon atoms.

3. The compound according to claim 1 or 2, wherein n is 1 or 2.

4. The compound according to claim 3, wherein, in the general formula [1], X and Y each independently represents a hydrogen atom, a fluorine atom, a trifluoromethyl group, a difluoromethyl group, or a methoxy group; R1, R3, R3 and R4 each represents a hydrogen atom; R5 represents an alkyl group having 1 to 5 carbon atoms, or an alkylene group which has 2 to 4 carbon atoms and binds to a carbon atom other than a carbon atom bound to a nitrogen

atom on the cyclohexyl ring to form a ring; and n is 1.

**5.** The compound according to claim 3 or 4, wherein, in the general formula [1], X is a hydrogen atom or a methoxy group; Y represents a hydrogen atom, a fluorine atom, a trifluoromethyl group, a difluoromethyl group, or a methoxy group; R1, R2, R3 and R4 each represents a hydrogen atom; R5 represents an alkyl group having 1 to 3 carbon atoms, or an alkylene group which has 2 to 4 carbon atoms and binds to a carbon atom other than a carbon atom bound to a nitrogen atom on the cyclohexyl ring to form a ring.

**6.** The compound according to claim 5, which is any one of the following compounds [2] to [12], or a pharmacologically acceptable salt thereof:

[2] N-(2-(phenylthio)ethyl)-N-ethylcyclohexylamine,
[3] N-(2-phenylthio)ethyldecahydroquinoline,
[4] N-[2-(3-trifluoromethyl)phenylthio]ethyl-N-ethylcyclohexyla mine,
[5] N-[2-(3-difluoromethyl)phenylthio]ethyl-N-ethylcyclohexylam ine,
[6] N-[2-(3,4-dimethoxy)phenylthio]ethyl-N-ethylcyclohexylamine
[7] N-[2-(4-fluorophenylthio)ethyl]-N-ethylcyclohexylamine,
[8] N-(3-phenylpropyl)-N-ethylcyclohexylamine,
[9] N-[3-(4-methoxyphenyl)propyl]-N-ethylcyclohexylamine,
[10] N-[3-(4-fluorophenyl)propyl]-N-ethylcyclohexylamine,
[11] N-ethyl-N-[2-(4-fluorophenoxy)ethyl]cyclohexylamine, and
[12] N-ethyl-N-(2-phenoxyethyl)cyclohexylamine.

**7.** A pharmaceutical composition for treatment or prevention of a disease accompanied by a central nervous system disorder, comprising as an active ingredient at least one of a compound represented by the following general formula (1') and a pharmacologically acceptable salts thereof:

(Chemical formula 2)

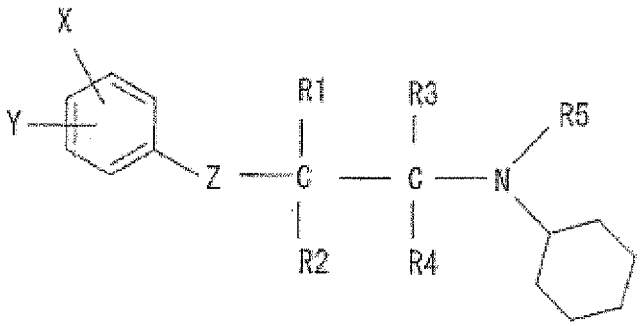

[1']

wherein X and Y each independently represents a hydrogen atom, a fluorine atom, a trifluoromethyl group, a difluoromethyl group, a monofluoromethyl group, a methoxy group, a hydroxyl group, or an alkyl group having 1 to 3 carbon atoms; R1, R2, R3 and R4 each independently represents a hydrogen atom, a hydroxyl group, an alkoxyl group having 1 to 3 carbon atoms, or an alkyl group having 1 to 3 carbon atoms; R5 represents a hydrogen atom, an alkyl group having 1 to 5 carbon atoms, or an alkylene group which has 2 to 4 carbon atoms and binds to a carbon atom other than a carbon atom bound to a nitrogen atom on the cyclohexyl ring to form a ring; and Z represents a sulfur atom, a carbon atom, or an oxygen atom.

**8.** The pharmaceutical composition according to claim 7, wherein, in the general formula (1'), X is a hydrogen atom or a methoxy group; Y represents a hydrogen atom, a fluorine atom, a trifluoromethyl group, a difluoromethyl group, or a methoxy group; R1, R2, R3 and R4 each represents a hydrogen atom; R2 represents an alkyl group having 1 to 3 carbon atoms, or an alkylene group which has 2 to 4 carbon atoms and binds to a carbon atom other than a carbon atom bound to a nitrogen atom on the cyclohexyl ring to form a ring.

**9.** The pharmaceutical composition according to claim 8, comprising as an active ingredient at least one of the following compounds [2], [3], [4], [5], [6], [7], [8], [9], [10], [11], and [12], and pharmacologically acceptable salts thereof:

[2] N-(2-(phenylthio)ethyl)-N-ethylcyclohexylamine,
[3] N-(2-phenylthio)ethyldecahydroquinoline,
[4] N-[2-(3-trifluoromethyl)phenylthio]ethyl-N-ethylcyclohexyla mine,
[5] N-[2-(3-difluoromethyl)phenylthio]ethyl-N-ethylcyclohexylam ine,
[6] N-[2-(3,4-dimethoxy)phenylthio]ethyl-N-ethylcyclohexylamine [7] N-[2-(4-fluorophenylthio)ethyl]-N-ethylcyclohexylamine,
[8] N-(3-phenylpropyl)-N-ethylcyclohexylamine,
[9] N-[3-(4-methoxyphenyl)propyl]-N-ethylcyclohexylamine,
[10] N-[3-(4-fluorophenyl)propyl]-N-ethylcyclohexylamine,
[11] N-ethyl-N-[2-(4-fluorophenoxy)ethyl]cyclohexylamine, and
[12] N-ethyl-N-(2-phenoxyethyl)cyclohexylamine.

**10.** The pharmaceutical composition according to claim 9, comprising as an active ingredient at least one of the following compounds [2], [3], [5], and [8], and pharmacologically acceptable salts thereof:

[2] N-(2-(phenylthio)ethyl)-N-ethylcyclohexylamine,
[3] N-(2-phenylthio)ethyldecahydroquinoline,
[5] N-(2-(3-difluoromethyl)phenylthio)ethyl-N-ethylcyclohexylam ine, and
[8] N-(3-phenylpropyl)-N-ethylcyclohexylamine.

**11.** The pharmaceutical composition according to any one of claims 7 to 10, wherein the disease accompanied by a central nervous system disorder is at least one selected from the group consisting of Alzheimer's disease, Parkinson's disease, Huntington's disease, sequelae of cranial neuropathy of cerebrovascular disorder, dementia, disease accompanied by cranial neuropathy due to head injury, and disease accompanied by motor dysfunction due to spinal cord injury.

**12.** A method for treatment or prevention of a disease accompanied by a central nervous system disorder, **characterized by** administering a therapeutically or preventively effective amount of at least one of the compound according to any one of claims 1 to 6 and a pharmacologically acceptable salt thereof, for the treatment or prevention of the disease accompanied by a central nervous system disorder, to a subject in need of said treatment or prevention.

**13.** The method according to claim 12, wherein the disease accompanied by a central nervous system disorder is at least one selected from the group consisting of Alzheimer's disease, Parkinson's disease, Huntington's disease, sequelae of cranial neuropathy due to cerebrovascular disorder, dementia, disease accompanied by cranial neuropathy due to head injury, and disease accompanied by motor dysfunction due to spinal cord injury.

**14.** Use of at least one of any one of the compounds according to claims 1 to 6 and a pharmacologically acceptable salt thereof as an active ingredient for the production of a pharmaceutical composition for treatment or prevention of a disease accompanied by a central nervous system disorder.

Fig. 1

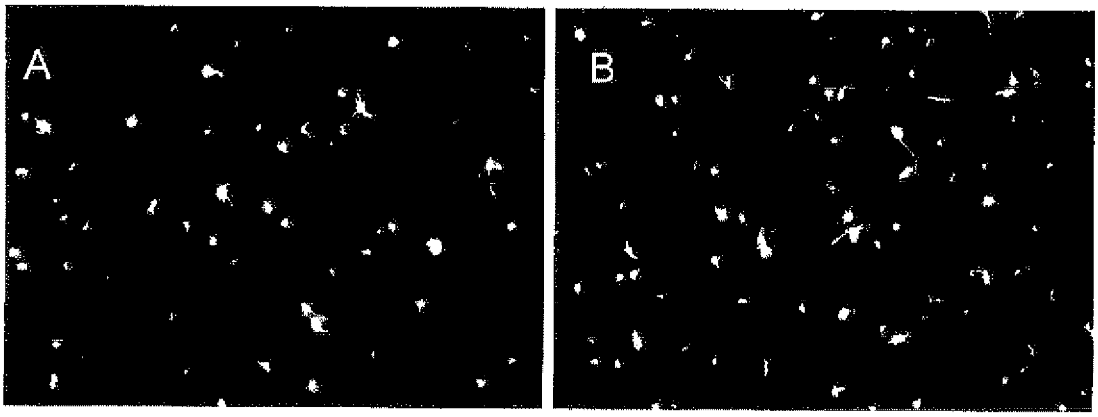


No additives

Compound [2]

Fig. 2

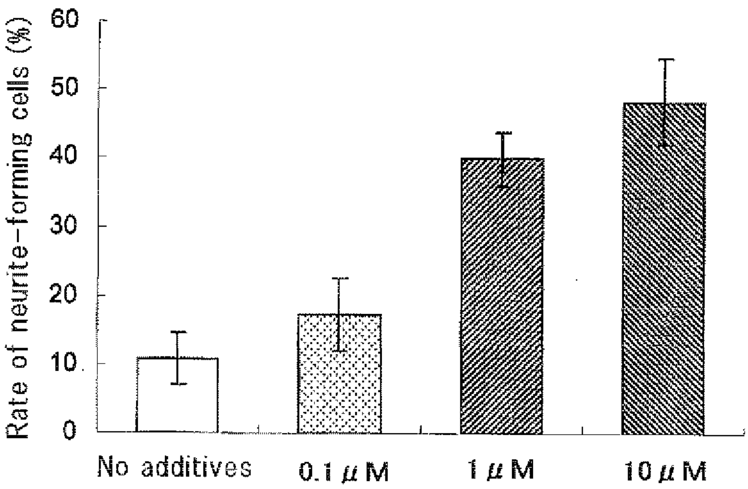

**INTERNATIONAL SEARCH REPORT**

International application No.
PCT/JP2009/068340

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07C211/40, A61K31/137, A61K31/138, A61K31/145, A61K31/47, A61P9/10, A61P25/00, A61P25/14, A61P25/16, A61P25/28, A61P43/00, C07C217/16, C07C217/20, C07C217/62, C07C323/25, C07D215/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | Charles N. SATTERFIELD and Shan Hsi YANG, Industrial & Engineering Chemistry Process Design and Development, 1984, Vol.23, No.1, p.11-19 | 1-5<br>6 |
| X | JP 1-501393 A (A/S Cheminova), 18 May 1989 (18.05.1989), claims; compounds 30, 41<br>& EP 269363 A2 & WO 1988/003756 A1<br>& DK 368687 A & NO 883244 A<br>& AU 8278687 A & BR 8707539 A | 1-6 |
| X<br>Y<br>A | R. D. Schoenwald et al., Journal of Ocular Pharmacology, 1993, Vol.9, No.2, p.125-139 | 1-5<br>7,8,11,14<br>6,9,10 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 November, 2009 (09.11.09) | 24 November, 2009 (24.11.09) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No.
PCT/JP2009/068340

C (Continuation). DOCUMENTS CONSIDERED TO BE RELEVANT

| Category* | Citation of document, with indication, where appropriate, of the relevant passages | Relevant to claim No. |
|---|---|---|
| X<br>Y<br>A | SATISH Shirolkar et al., Journal of Ocular Pharmacology, 1993, Vol.9, No.3, p.211-227 | 1-5<br>7,8,11,14<br>6,9,10 |
| X<br>Y<br>A | US 5387614 A (University of Iowa Research Foundation),<br>07 February 1995 (07.02.1995),<br>full descriptions<br>(Family: none) | 1-5<br>7,8,11,14<br>6,9,10 |
| X | PL 164665 B1 (Politechnika Szczecinska),<br>30 September 1994 (30.09.1994),<br>table 1; compound 3<br>(Family: none) | 1-6 |
| X<br>A | JP 8-283402 A (Kissei Pharmaceutical Co., Ltd.),<br>29 October 1996 (29.10.1996),<br>example 4<br>(Family: none) | 1-3<br>4-6 |
| X<br>A | Kunio HIROI et al., Tetrahedron: Asymmetry, 1998, Vol.9, No.21, p.3797-3817 | 1-3<br>4-6 |
| X<br>A | US 2008/0194630 A1 (William T. BARCHUK et al.),<br>14 August 2008 (14.08.2008),<br>paragraph [0702]<br>& WO 2008/100564 A1 & UY 30918 A<br>& CL 4682008 A & AR 65355 A<br>& PE 4872009 A | 1-3<br>4-6 |
| Y | JP 2007-500757 A (M's Science Corp.),<br>18 January 2007 (18.01.2007),<br>paragraph [0006]<br>& JP 2009-516650 A & US 2005/0020483 A1<br>& US 2005/0032827 A1 & US 2005/0059689 A1<br>& US 2006/0014753 A1 & US 2006/0052386 A1<br>& EP 1635805 A & EP 1644026 A<br>& EP 1951241 A & WO 2004/110387 A2<br>& WO 2004/110388 A2 & WO 2004/110389 A2<br>& WO 2007/053580 A2 & CA 2528160 A<br>& CN 1802168 A & CA 2621985 A | 7,8,11,14 |

Form PCT/ISA/210 (continuation of second sheet) (April 2007)

**INTERNATIONAL SEARCH REPORT**

International application No.
PCT/JP2009/068340

Continuation of A. CLASSIFICATION OF SUBJECT MATTER
(International Patent Classification (IPC))

*C07C211/40*(2006.01)i, *A61K31/137*(2006.01)i, *A61K31/138*(2006.01)i, *A61K31/145*(2006.01)i, *A61K31/47*(2006.01)i, *A61P9/10*(2006.01)i, *A61P25/00*(2006.01)i, *A61P25/14*(2006.01)i, *A61P25/16*(2006.01)i, *A61P25/28*(2006.01)i, *A61P43/00*(2006.01)i, *C07C217/16*(2006.01)i, *C07C217/20*(2006.01)i, *C07C217/62*(2006.01)i, *C07C323/25*(2006.01)i, *C07D215/06*(2006.01)i

(According to International Patent Classification (IPC) or to both national classification and IPC)

Form PCT/ISA/210 (extra sheet) (April 2007)

**INTERNATIONAL SEARCH REPORT**

International application No.
PCT/JP2009/068340

**Box No. II Observations where certain claims were found unsearchable (Continuation of item 2 of first sheet)**

This international search report has not been established in respect of certain claims under Article 17(2)(a) for the following reasons:

1. [X] Claims Nos.: 12, 13
because they relate to subject matter not required to be searched by this Authority, namely:
Claims 12 and 13 pertain to methods for treatment of the human body by therapy and thus relate to a subject matter which this International Searching Authority is not required, under the provisions of Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2007)

**INTERNATIONAL SEARCH REPORT**

International application No.
PCT/JP2009/068340

<Subject of search>

It is described that the carbon atom which is contained in the compound represented by general formula [1] recited in claim 1 and to which R1 and R2 are bound has a valency of 6.

However, this statement is technically wrong. Therefore, with respect to claim 1 and parts of claims 2-5 and 14 which refer to claim 1, the search was made under the understanding that the moiety represented by "$-(CH_2)_n-$"is correctly to be -- $-(C)_n-$ --.

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20050020483 A
- US 5739158 A